# EUROPEAN PATENT SPECIFICATION

(11) **EP 0 667 767 B1**
(45) Date of publication and mention of the grant of the patent: **15.03.2000**
(21) Application number: 94900216.6
(22) Date of filing: 16.11.1993
(51) Int. Cl.: A61K 9/107, A61K 9/127

(54) **METHOD FOR THE PREPARATION OF CREAMS**
VERFAHREN ZUR HERSTELLUNG VON CREMES
PROCEDE DE PREPARATION DE CREMES

(30) Priority: 16.11.1992 GB 9224010
(43) Date of publication of application: 23.08.1995
(73) Proprietor: PHARES PHARMACEUTICAL RESEARCH N.V., Curacao Netherlands Antilles (AN)
(72) Inventor: LEIGH, Steven, Stevens, Hewlett & Perkins, London EC4Y 1LL (GB)
(74) Representative: Gaunt, Robert John
(86) International application number: GB9302355
(87) International publication number: WO9410978

(56) References cited:
- EP-A- 0 042 076
- EP-A- 0 084 341
- EP-A- 0 256 821
- EP-A- 0 424 282
- EP-A- 0 474 270
- EP-A- 0 523 418
- DE-A- 3 042 975
- H. JANISTYN 'HANDBUCH DER KOSMETIKA UND RIECHSTOFFE VOL1 THIRD EDITION' 1978 , DR. ALFRED HÜTHIG VERLAG GMBH , HEIDELBERG (DE) SEE PAGE 566 , "LECITHIN-PRODUKT M-C-88"

## Description

This invention relates to emulsifying compositions and emulsions formed therefrom, based on membrane lipids, chiefly phospholipids and glycolipids.

Emulsions and creams are dispersions of two immiscible phases either as oil droplets dispersed in water (O/W) or water globules suspended in oil (W/O). There are two important considerations to emulsion formation.
i) Work energy has to be applied to obtain dispersed droplets.
ii) Surfactants are required to lower interfacial tension and prevent the globules from coalescing.
These considerations lead to the use of emulsifying agents most of which are surfactants. Surfactants are normally amphiphiles, which are compounds that have a water-soluble (hydrophilic) and an oil-soluble (lipophilic) moiety. It should be emphasised, however, that not all amphiphiles are efficient emulsifying agents. Empirically, emulsifying agents are classed according to their affinity for water and oil in terms of their hydrophile-lipophile-balance (HLB). This is denoted by an arbitrary value from 1 upwards. Emulsifying agents with low HLB values (1 to about 8) form W/0 systems, whereas those with higher HLB values give rise to 0/W systems. It is also recognised that the irritancy of surfactants often increases with the HLB number.

Primary emulsifying agents are usually high HLB surfactants that form stable emulsions or dispersions of oil droplets in water. However, auxiliary or co-emulsifiers are frequently included to help further stabilise the dispersed droplets, although co-emulsifiers on their own do not form stable emulsions.

Emulsifying agents are widely used in the food, cosmetic, toiletry and pharmaceutical industries to form stable dispersions of oil and water. Many types of emulsifying agents are available to obtain W/O and O/W dispersions. With few exceptions, the most commonly used emulsifying agents are all synthetic compounds based on soaps of fatty acids (anionic), quarternary ammonium compounds (cationic), condensation products of ethylene oxide with sugars, glycol esters and ethers, fatty acid esters and ethers (non ionic).

EP-A-0424282 describes creams in the form of o/w emulsions comprising liposomes, a membrane lipid, a C₁₂ - C₂₂ fatty acid alcohol, glycerine and water. The cream bases quoted in the examples contain synthetic emulsifiers.

There is growing evidence that synthetic emulsifying agents frequently give rise to toxicity and irritancy problems. Moreover, in many cases, manufacture of these compounds often involve environmentally harmful solvents and residues.

Phospholipids are essential components of all cell membranes. In addition to being biocompatible, they are also thought to have useful physical and physiological functions. Therefore, phospholipids are widely used in all types of preparations intended for human use. It is well known to those skilled in the art that on their own phospholipids are poor primary emulsifying agents for O/W systems. This is due to the inherently low HLB values of natural phospholipids which vary between 3 to 8, depending on the type of phospholipid. To overcome the problem BP 1163227 describes emulsifying agents which contain hydroxylated phospholipids modified to improve water dispersibility. Dispersions of oil-in-water relying on phospholipids as the sole emulsifier are liable to cream and phase separate on standing, even after homogenisation. Coalescence of smaller globules into larger droplets results in creaming, leading to phase separation (cracking) if the process continues.

Phospholipids are, however, conventionally employed as auxiliary emulsifiers with more efficient anionic, cationic or non-ionic emulsifying agents with higher HLB numbers, to prepare O/W emulsions and creams. Thus, GB-A-1360021 discloses emulsifying agents based on phospholipids with glyceryl mono stearate and sodium stearate which is a soap with a very high HLB value.

Where phospholipids are used as sole O/W emulsifying agent, it is necessary to employ very high energy to obtain small oil droplets (below lu) which are more stable due to their smaller surface area. High energy processing requires generation of pressures in the order of 20000 psi and the equipment is expensive and not generally available. Therefore this method is confined to the production of intravenous fat emulsions in total parenteral nutrition (TPN). The resultant emulsion lacks the consistency of a cream, often described as 'body'.

EP-A-0084341 discloses an emulsion-type composition for external use containing lecithin and a polyhydric alcohol/fatty acid ester. The composition may be of either oil-in-water or water-in-oil type.

Recently, liposomes - vesicular structures prepared from membrane lipids - have found increasing favour as carriers of biologically active compounds. Frequently there is need to blend liposomes which may contain biologically active compounds into creams and emulsions, to increase the range of applications and extend their utility. However, it is now known to workers in liposome technology that relatively small amounts of synthetic surfactants that are necessary to form stable O/W emulsions can perturb the liposome membrane and cause leakage and lysis. In the November 1992 issue of INFORM featuring LIPOSOMES, it is stated that:
"In liposomal formulations, care must be taken to avoid using surfactants and materials that can act as a co-solvent for the phospholipid or breakdown the bilayers......Creams containing traditional phospholipid based liposomes are relatively difficult to formulate because the emulsion may separate or the liposome may breakdown when in contact with the oil phase.....it is important that companies extensively test their products to make sure the liposomes stay intact after formulation before making claims that a product contains liposomes."
This is not yet generally recognised by manufacturers and presently there are claims to liposome-presence in commercial products that contain damaging synthetic emulsifying agents which cause destruction of liposomes.

Liposome structure is usually confirmed through freeze-fracture electron microscopy and retention studies using specialised equipment and such facilities are not generally available. There is therefore an urgent need for a system of emulsification to form creams that could be blended with liposomes to obtain a liposome-containing cream in which the presence of liposomes can be verified.

The present invention is in connection with emulsifying systems which are compositions based essentially on membrane lipids, particularly phospholipids, in combination with at least one other natural amphiphile which is not an efficient primary emulsifier, ie does not form a stable emulsion by itself. It seeks to avoid the use of synthetic anionic, cationic and non-ionic surfactants with high or low HLB values to produce stable emulsions and creams, particularly of the oil-in-water type. The compositions are ideally suited for forming liposome-containing creams and emulsions. It is a further advantage of the system that relatively small proportions of phospholipids are required to obtain stable emulsions without recourse to excessive energy input.

The invention results from the unexpected discovery that combinations of membrane lipid and at least one amphiphile which is not a primary emulsifier (high HLB) can form stable dispersions of oil droplets in water. In the prior art, membrane lipids are mostly combined with surfactants having high HLB values to obtain stable 0/W emulsions.

In one aspect, the invention relates to the use of an emulsifier composition for forming oil-in-water creams and which comprises:
a) at least one membrane lipid,
b) at least one natural amphiphile which is not a primary emulsifier, and
c) a hydrophilic medium,
   with the proviso that synthetic emulsifiers or surfactants are absent from the said composition.

In another aspect, the invention relates to the use of an emulsifier composition for forming oil-in-water creams and which consists of
a) at least one membrane lipid, and
b) at least one natural amphiphile which is not a primary emulsifier,
   with the provise that synthetic emulsifiers or surfactants are absent from the said composition.

These compositions are highly efficient emulsifying systems and are characterised by the absence of synthetic emulsifiers.

In a second aspect, these combinations form stable O/W creams with oils and oily substances. The dispersion of oil globules in the resultant cream is mostly below 5 u and is obtained without synthetic surfactants with high HLB values.

In a third aspect, the invention also provides a stable emulsion or a cream which may be blended with liposomes. The liposomes may be prepared by any method, eg, sonication, extrusion, reverse phase evaporation, high shear mixing. However, it is preferred to incorporate liposomes which have high entrapped volumes i.e., 6 to 10 mls/gm lipid, prepared in accordance with the pro-liposome method described in EP 0158441.

Examples of component a) are phospholipids such as phosphatidylcholine, phosphatidyl ethanolamine, phosphatidyl inositol, phosphatidyl serine, phosphatidic acid, mixtures of phospholipids known commercially as lecithin and glycolipids eg. cerebrocides and gangliocides. The lipids may be saturated, partially saturated or unsaturated. Unsaturated lipids are prone to oxidation and it is often advantageous to use saturated lipids to give more stable compounds. The hydrocarbon region may be fully or partially saturated, depending on the degree of hydrogenation. This specification covers the use of unsaturated, partially saturated and fully saturated lipid obtained from plant or animal sources. The compositions comprise 5% to 95%, optimally between 10% to 50% of component a) which is preferably a lecithin. Preferably component b) is a fatty acid or alcohol of chain length C₁₂ to C₂₂, such as lauric, palmitic stearic and behenic acids, cetyl, stearyl and cetostearyl alcohols, or even longer chain compounds (i.e. C₂₄ and above). Alternative amphipaths such as cholesterol, sitosterol and phytosterol may also be used in special circumstances. Combinations of fatty acid and alcohol, and chain lengths of between C₁₂ and C₂₂, are particularly preferred. The proportion of component b) is between 95% to 5%, preferably between 10% to 90%, ideally between 40% to 80%. Conventionally, fatty acids and alcohols are used in combination with high HLB surfactants eg, cetomacrogol emulsifying wax. The reason they form efficient 0/W emulsifiers in combination with phospholipids which are poor emulsifiers may be due to formation of bilayered vesicular structures in the aqueous phase, some of which contain and segregate the oil droplets. When liposomes are added to the creams in one embodiment of the invention, some reorganising of the bilayers take place. The final proportion of phospholipid to fatty acid/alcohols in liposome-containing creams should be between 6:1 and 1:6, preferably not less than 1:3.

The vesicular structures present in the creams prepared by this emulsifying system contain aqueous spaces and the entrapped volumes given by inulin inaccessible space determination range from about 20% to over 60% of the total water present in the creams.

Optionally, the composition may also contain minor proportions, up to 25% of c), a hydrophilic medium comprising an aliphatic alcohol eg. ethanol, isopropyl alcohol, propylene glycol and glycerol, singly or in combination. The hydrophilic medium is not essential for emulsification. It mainly helps to dissolve the components.

The emulsifying compositions disclosed herein give stable dispersions of oil globules. The oil may be a vegetable triglyceride, medium chain triglyceride, mono or polyhydric alcohol ester, branched chain alcohol, paraffin, squalane or any water immiscible substance that is conventionally referred to as an oil or waxy material. In some cases, an oil soluble biologically active compound can be dissolved in the oil.
The ratio of emulsifying agent to oil is between 1:0.5 to 1:20, preferably between 1:1 to 1:10, ideally between 1:2 to 1:8. In forming oil-in-water dispersions, the oily phase comprising oil and emulsifiers should constitute between 5% and 60% of the total, whilst the continuous aqueous phase comprises from 40% to 95%. Above approximately 50% of oil, phase inversion could lead to a water-in-oil system.

In practice, a number of factors have to be optimised to obtain the most stable dispersion. Variables such as membrane lipid and its concentration, fatty acid/alcohol mixture, ratio of emulsifier to oil, type of oil, level of oil, have all to be taken into account.

By optimising the parameters, stable O/W and in some cases W/O dispersions can be obtained without requiring synthetic surfactants. Furthermore, the resultant creams are carriers of liposomes with or without biologically active compounds. These may be present in the oil globules, the continuous aqueous phase, or entrapped in the liposomes.

Stability and quality of the creams can be assessed by observing the rate of creaming or phase separation after storage and or centrifugation at medium speeds (2000 to 5000 rpm). Stable preparations should show minimum creaming and no phase separation.

Assessment of compatibility between the cream and liposomes was carried out as follows.

Liposome integrity and structure were determined by means of inulin inaccessible space measurements and freeze fracture electron microscopy.

Entrapped volume was determined by adding a precise amount of labelled inulin to a known suspension of liposome containing cream, followed by centrifugation at 100000 g to separate the liposomes from the cream. If the liposomes were intact, the entrapped aqueous space should be impermeable to inulin. Therefore, the concentration of labelled inulin recovered in the supernatant after allowance for the known volume of the emulsified oil present in the cream gave a measure of the internal volume. Liposome structure was further confirmed by means of freeze fracture electron microscopy through identification of bilayers across the fracture plane.

The following examples describe the invention in greater detail. 1 to 4 are typical examples of emulsifying systems disclosed in this specification. Formulations 5 to 8 are stable creams and emulsions which contain varying proportions of an oil stabilised with the system disclosed and not relying on synthetic emulsifiers. Example 9 is a typical formulation of a liposome-containing cream with added liposomes, according to the invention.

### EMULSIFYING COMPOSITIONS

### Example 1

| | |
|---|---|
| Hydrogenated soya bean lecithin | 50% |
| Cetyl alcohol | 50% |

The 2 components were co-dissolved at 80 to 85 C to obtain a uniform mixture. This mixture set to a hard wax at room temperature. The composition may be used as such or powdered.

### Example 2

| | |
|---|---|
| Hydrogenated soya bean lecithin | 20% |
| Palmitic acid | 20% |
| Cetyl alcohol | 60% |

The 3 components were melted at 80 to 85 C to obtain a homogeneous emulsifying composition, which is a hard wax at room temperature. As in example 1, the composition may be pulverised or spray congealed into small droplets or free flowing powders. The hydrogenated lecithin can be replaced with unsaturated or partially saturated lecithin.

### Example 3

| | |
|---|---|
| Hydrogenated soya bean lecithin | 15% |
| Lauric acid | 15% |
| Glycerol | 15% |
| Cetostearyl alcohol | 55% |

The 4 components were heated to about 80 C until a uniform composition was obtained, solidifying to a waxy solid at room temperature.

### Example 4

| | |
|---|---|
| Hydrogenated soya bean lecithin | 15% |
| Palmitic acid | 15% |
| Ethanol | 10% |
| Glycerol | 15% |
| Cetyl alcohol | 45% |

The solids were dissolved in the mixture of ethanol and glycerol at 65 to 70 C, in a covered container to prevent loss of alcohol. A soft waxy mass formed on cooling. In the above examples, the saturated phospholipids can be replaced by unsaturated or partially saturated lipids.

### O/W CREAMS

### Examples 5,6,7 & 8

| | **10%** | **20%** | **30%** | **40%** |
|---|---|---|---|---|
| Emulsifying composition | 5 | 5 | 5 | 5 |
| Oil | 10 | 20 | 30 | 40 |
| Distilled water | 85 | 75 | 65 | 55 |

The examples are typical formulations.
The emulsifying base was prepared according to Example 2. In each case , it was melted in the oil at about 65 to 75 C, depending on the oil.
Distilled water at a similar temperature was gradually added with stirring. It is to be understood that this specification also covers creams that are prepared by simply incorporating the components typically disclosed in examples 1 to 4 separately. It is only necessary to have in the finished cream preparation the appropriate phospholipids and fatty acids/alcohols in the correct proportions.

The resultant creams can be homogenised using eg. a high speed stirrer, colloid mill, or any suitable homogeniser. Smooth, structured O/W creams were obtained on cooling.

Examples 5 to 8 may also be prepared with any of the other emulsifiers given in Examples 1 to 4.

The proportion of lipid to oil and the level of oil can be varied up or down to obtain lotions or full-bodied creams. However, levels of oil above 40% tend to give rise to W/O systems. The type of oil used could affect the viscosity and rheology of the final product, and therefore the proportion of emulsifier may have to be adjusted to give a cream of the required consistency. Blends of oils can be used. Lipophilic compounds eg. cholesterol & phytosterol may also be included in the formulations to modify the physical properties without affecting stability. Biologically active lipophilic compounds may additionally be dissolved in the oil.

The creams according to this invention are characterised by the absence of synthetic emulsifiers. It should be noted that although it is neither necessary nor preferred, in some cases minor proportions of these substances can be included to modify the rheology of the creams. Viscosity improving gums and thickeners conventionally included in creams may also be added in small amounts, usually not more than 1%.

### LIPOSOME CREAMS

### Example 9

| | |
|---|---|
| Cream (Examples 5 to 9) | 60% |
| Liposome suspension (with 50% entrapped volume) | 40% |

The cream was prepared in accordance with Example 5 to 9. The liposome suspension, with or without a biologically active compound was prepared using Lucas Meyer's PRO-LIPO, pro-liposome composition.

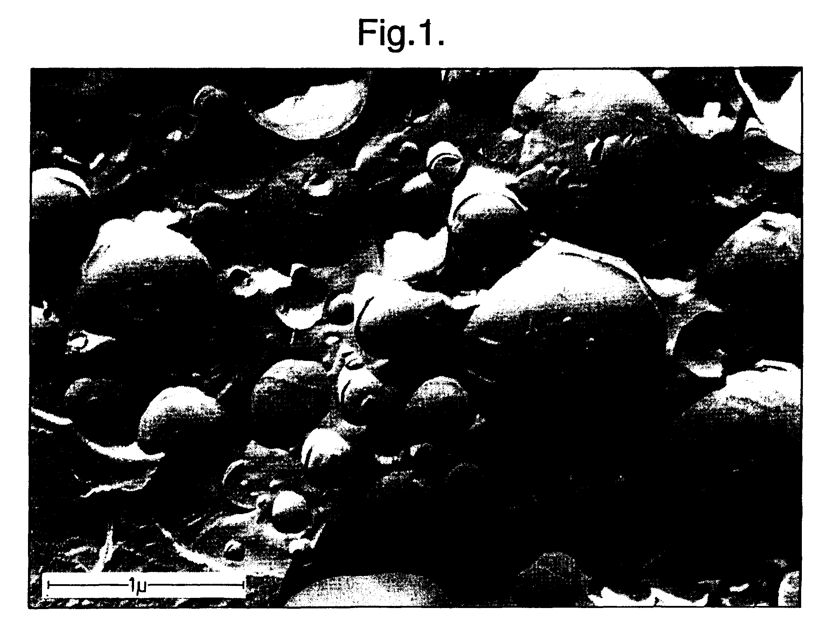

The liposome suspension was blended in with the cream with simple stirring to obtain a liposome cream. Alternatively, a pro-liposome composition may be added, so that conversion to liposomes occurs in situ in the continuous phase of the cream. In any event, it is important that the lipid to fatty acid/alcohol ratio in the liposome containing cream is between 6:1 and 1:6, preferably not less than 1 part of lipid to 3 parts of fatty acid/alcohol. The oil content should preferably be between 10% to 20% of the total. Electron micrographs of the liposome pellet separated from a liposome cream is shown in Fig 1.

It should be noted that liposome containing creams may also be formed in situ by;
i. Dispersing 5 to 10 parts of a composition shown in examples 1 to 4 in 60 to 90 parts of water to obtain an intermediate oil-free preparation using a suitable production apparatus with mixing and heating facilities.
ii. Adding the pro-liposome or liposome component (Example 9), preservatives and perfumes to make up 100 parts.
iii. Packing into smaller containers for use by the end users.
iv. Applying the composition contained in (iii) to the skin, mopping up (emulsifying) oily secretions and grease by rubbing, and at the same time forming a cream in situ.
v. Optionally, rinsing off with water when further emulsification and rearrangement of the bilayers occur leaving liposomes on the skin surface.

This specification describes emulsifying systems containing membrane lipids and natural amphiphiles and the resultant creams prepared without the aid of synthetic emulsifying agents.

In one embodiment, it discloses emulsifying compositions comprising at least one membrane lipid together with at least one other natural amphiphile which is not a primary emulsifier.

In a second embodiment, it relates to preparations of dispersed oil droplets in water, containing natural amphiphiles and not requiring synthetic emulsifiers to form stable emulsions and creams.

In a further embodiment, it is a liposome-containing cream prepared with an emulsifying system comprising membrane lipids and fatty acids/alcohols, to which liposomes or liposome forming pro-liposome compositions are added.

## Claims

1. Use of An emulsifier composition for forming oil-in-water creams and which comprises:-
a) at least one membrane lipid,
b) at least one natural amphiphile which is not a primary emulsifier,
and,
c) a hydrophilic medium,
with the proviso that synthetic emulsifiers or surfactants are absent from the said composition.

2. Use of An emulsifier composition for forming oil-in-water creams and which consists of:-
a) at least one membrane lipid, and
b) at least one natural amphiphile which is not a primary emulsifier,
with the proviso that synthetic emulsifiers or surfactants are absent from the said composition.

3. Use as claimed in claim 1 or claim 2, wherein the membrane lipid is a phospholipid.

4. Use as claimed in any of the preceding claims, wherein the natural amphiphile component comprises a C₁₂ to C₂₂ fatty acid or alcohol.

5. Use as claimed in claims 1 to 3, wherein the natural amphiphile component comprises a mixture of C₁₂ to C₂₂ fatty acids and alcohols.

6. Use as claimed in any of the preceding claims, wherein the hydrophilic medium comprises one or more aliphatic alcohols.

7. Use as claimed in any of the preceding claims, which comprises a) from 10 to 50% by weight of at least one membrane lipid and b) from 90 to 50% by weight of at least one natural amphiphile.

8. Use as claimed in any of the preceding claims, wherein the hydrophilic medium of component c) is present in an amount of up to 25% by weight.

9. Use as claimed in any of claims 1 to 8, wherein the said oil-in-water cream is in the form of An oil-in-water emulsion or dispersion in which the mean diameter of the oil droplets or globules in the emulsion or dispersion is within the range of from 1µ to 5µ.

10. Use as claimed in any of claims 1 to 9, wherein the said oil-in-water cream comprises bilayered structures segregating oil droplets and having entrapped volumes of at least about 20% of the total aqueous phase.

11. Use as claimed in any of claims 1 to 10, that further involves adding oil to an emulsifier composition and dispersing the resulting mixture in an aqueous medium so as to form said emulsion or dispersion.

12. Use as claimed in any of claims 1 to 8, that further involves adding oil to, or adding to oil, the said emulsifier composition and an aqueous phase, so as to form the said oil-in-water cream.

13. Use as claimed in any of claims 1 to 12, wherein the oil-in-water cream also contains liposomes.

14. Use as claimed in claim 13, wherein there are present one or more biologically active compounds.

15. Use as claimed in claim 14, wherein the one or more biologically active compounds are present in the oil globules and/or the continuous aqueous phase and/or are entrapped within the lipid vesicles or liposomes.

16. Use as claimed in any of claims 13 to 15, wherein the oil-in-water emulsion or dispersion is blended together with lipid vesicles or liposomes, or precursor components thereof, and, optionally, one or more biologically active compounds.

## Patentansprüche

1. Verwenden einer Emulgierzusammensetzung, um Öl-in-Wasser Cremes zu entwickeln, die enthalten:-
a) mindestens ein Membranlipid,
b) mindestens ein natürliches Amphiphil, das kein Primäremulgiermittel ist,
und
c) ein wasserliebendes Lösungsmittel
mit dem Vorbehalt, daß in der genannten Zusammensetzung keine synthetischen Emulgiermittel oder oberflächenaktiven Substanzen enthalten sind.

2. Verwenden einer Emulgierzusammensetzung für das Entwickeln von öl-in-Wasser-Cremes, bestehend aus:
a) mindestens einem Membranlipid, und
d) mindestens einem natürliches Amphiphil, das kein Primäremulgiermittel ist,
mit dem Vorbehalt, daß in der genannten Zusammensetzung keine synthetischen Emulgiermittel oder oberflächenaktiven Substanzen enthalten sind.

3. Verwenden wie in Anspruch 1 oder 2 aufgeführt, wobei die Membranlipiden Phosphilipiden sind.

4. Verwenden wie in den vorangegangenen Ansprüchen, wobei der natürliche Amphiphilanteil eine C12 bis C22 Fettsäure oder Alkohol enthält.

5. Verwenden wie in den Ansprüchen 1 bis 3, wobei der natürliche Amphiphilanteil eine Mischung aus C12 bis C22 Fettsäuren und Alkohol enthält.

6. Verwenden wie in einer der vorangegangenen Ansprüche, wobei das wasserliebende Lösungsmittel ein oder mehr aliphatische Alkohole enthält.

7. Verwenden wie in einem der vorangegangenen Ansprüche, wobei die Zusammensetzung besteht aus a) vom 10 bis 50% nach Gewicht von mindestens einem Membranlipid und b) 90 bis 50% nach Gewicht von mindestens einem natürlichen Amphiphil.

8. Verwenden wie in einem der vorangegangenen Ansprüche, wobei das wasserliebende Lösungsmittel des Bestandteils c) mit einem Anteil von bis zu 25% nach Gewicht vorhanden ist.

9. Verwenden wie in einem der Ansprüche 1 bis 8, wobei die benannte Öl-in-Wasser Creme die Form einer Öl-in-Wasser Emulsion hat oder wenn die Feinverteilung, in welcher der mittlere Durchmesser der Öltröpfchen oder die Kügelchen in der Emulsion oder in der Feinverteilung innerhalb des Bereichs von 1u bis 5u liegt.

10. Verwenden wie in einem der Ansprüche 1 bis 9, wobei die benannte Öl-in-Wasser Creme doppellagige Strukturen enthält, welche die Öltröpfchen trennen und die mindestens circa 20% eingeschlossenes Volumen der gesamten wasserhaltigen Phase hat.

11. Verwenden wie in einem der Ansprüche 1 bis 10, was weiterhin involviert, daß Öl einer Emulgierzusammensetzung hinzugefügt wird und daß die sich ergebende Mischung in einem wasserhaltigen Lösungsmittel zerteilt wird, damit die genannte Emulsion oder Feinverteilung entwickelt werden kann.

12. Verwenden wie in einem der Ansprüche 1 bis 8, was weiterhin involviert, daß Öl zu der genannten Emulgierzusammensetzung und eine wasserhaltige Phase oder diese zu Öl hinzugefügt werden, damit die genannte Öl-in-Wasser Creme entwickelt werden kann.

13. Verwenden wie in einem der Ansprüche 1 bis 12, wobei die Öl-in-Wasser Creme gleichzeitig Liposomen enthält.

14. Verwenden wie in Anspruch 13, wobei ein oder mehr biologisch aktive Zusammensetzungen vorhanden sind.

15. Verwenden wie in Anspruch 14, wobei ein oder mehr biologisch aktive Zusammensetzungen in den Ölkügelchen und/oder in der fortlaufenden wasserhaltigen Phase vorhanden sind und in den Lipidenbläschen oder Liposomen eingeschlossen sind.

16. Verwenden wie in den Ansprüchen 13 bis 15, wobei die Öl-in-Wasser Emulsion oder die Feinverteilung mit Lipidbläschen oder Liposomen oder mit den Vorstufenzusammensetzungen aus diesen zusammengemischt werden und wahlweise ein oder mehr biologisch aktive Zusammensetzungen.

## Revendications

1. Utiliser une préparation émulsifiante pour former des crèmes de type aqueux qui comprennent:-
a) au moins une membrane lipide,
b) au moins un amphiphile naturel qui n'est pas un émulsifiant primaire,
et
c) un milieu hydrophile.
avec comme condition que les émulsifiants sythétiques ou tensio-actifs soient absents de la dite préparation.

2. Utiliser une préparation émulsifiante pour former des crèmes de type aqueux et qui consiste en:-
a) au moins une membrane lipide, et
b) au moins un amphiphile naturel qui n'est pas un émulsifiant primaire,
avec comme condition que les émulsifiants synthétiques ou tensio-actifs soient absents de la dite préparation.

3. Utiliser une préparation comme affirmée dans la revindication 1 ou 2, dans laquelle la membrane lipide est un phospholipide.

4. Utiliser une préparation comme affirmée dans n'importe quelle des revendications précédentes dans laquelle le composant amphiphile naturel comprend un acide gras C12 à C22 ou un alcool.

5. Utiliser une préparation comme affirmée dans les revendications 1 á 3 dans laquelle le composé amphiphile naturel comprend un mélange d'acides gras C12 á C22 et des alcools.

6. Utiliser une préparation comme affirmée dans n'importe quelle des revendications précédentes, dans laquelle le milieu hydrophile comprend un ou plus d'acides aliphatiques.

7. Utiliser une préparation comme affirmée dans n'importe quelle des revendications précédentes, dans laquelle la préparation comprend a) une quantité de 20 à 50% d'au moins une membrane lipide et b) de 90 à 50% d'au moins un amphiphile naturel.

8. Utiliser une préparation comme affirmée dans n'importe quelle des revendications, dans laquelle le milieu hydrophile du composant c) est présent dans une quantité allant jusqu'à 25% en poids.

9. Utiliser une préparation comme affirmée dans n'importe quelle des revendications 1 à 8, dans laquelle, la dite crème de type aqueux est sous la forme d'une émulsion ou une dispersion de type aqueux dans laquelle le diamètre moyen des gouttelettes d'huile ou globules dans l'émulsion ou dispersion varie d'environ 1µ à 5µ.

10. Utiliser une préparation comme affirmée dans n'importe quelle des revendications 1 à 9, dans laquelle la dite crème de type aqueux comprend des structures à double couche séparant les gouttelettes d'huile d'au moins environ 20% de la phase totale aqueuse.

11. Utiliser une préparation comme affirmée dans les revendications 1 à 10, qui nécessite en plus d'ajouter de l'huile à une préparation émulsifiante et de disperser le mélange en résultant dans un mileu hydrophile afin de former la dite émulsion ou dispersion.

12. Utiliser une préparation comme affirmée dans les revendications 1 à 8, qui nécessite en plus d'ajouter de l'huile ou d'ajouter à l'huile, la dite préparation émulsifiante et une phase aqueuse, afin de former la dite crème de type aqueux.

13. Utiliser la préparation comme affirmée dans n'importe quelle des revendications 1 à 12, dans laquelle la crème de type aqueux contient aussi des liposomes.

14. Utiliser la préparation comme affirmée dans la revendication 13, dans laquelle il y a un ou plus d'un composants biologiquement actifs.

15. Utiliser la préparation commme affirmée dans la revendication 14, dans laquelle il y a un ou plus d'un composants biologiquement actifs dans les globules d'huile et/ou dans la phase aqueuse et/ou sont contenus à l'interieur des vésicules lipidiques ou liposomes.

16. Utiliser la préparation commme affirmée dans n'importe quelle des revendications 13 à 15, dans laquelle l'émulsion ou dispersion de type aqueux forme un mélange avec des vésicules lipidiques ou liposomes, ou composants précurseurs de cela, et, en option, un ou plus d'un composants actifs biologiquement.
